# EUROPEAN PATENT APPLICATION

(11) **EP 2 583 719 A1**
(43) Date of publication of application: **24.04.2013**
(21) Application number: 12172783.8
(22) Date of filing: 20.06.2012
(51) Int. Cl.: A61N 5/06, A61F 9/008, A61B 3/12

(54) **Diagnostic imaging for age-related macular degeneration (AMD) using second harmonic generation (SHG) techniques**

(30) Priority: 20.10.2011 US 201113278012
(71) Applicant: Heidelberg Engineering GmbH, 69121 Heidelberg (DE)
(72) Inventor: Bille, Joseph, 69118 Heidelberg (DE)
(74) Representative: Gates, Marie Christina Esther

(57) **Abstract**

A system for treating age-related macular degeneration includes an agent with non-centro symmetric molecules_for marking a region of diseased tissue. An optical assembly focuses the laser beam to a plurality of focal points in the region of diseased tissue, each focal point having a volumetric measurement of about 2µm x 2µm x 20µm. Due to an increased concentration of photons in the relatively small volume of each focal point, two photons interact with a single molecule of the marking agent, within a very short interval of time (e.g. 10⁻¹³ sec). The resultant excited electron state (e.g. 3eV) is sufficient to induce the marking agent to convert oxygen in a manner that causes the oxygen to kill the diseased tissue. Also, an interaction between photons and a non-centro symmetric molecule in the marking agent will cause a Second Harmonic Generation (SHG) response that can be used for imaging purposes.

## Description

### FIELD OF THE INVENTION

The present invention pertains generally to the treatment of disease in the retina of a human eye. More particularly the present invention pertains to the optical diagnosis and treatment of age-related macular degeneration. The present invention is particularly, but not exclusively, useful as a system and method for photodynamic therapy, characterized by using two-photon excitation, for the treatment of age-related macular degeneration in the retina of a human eye.

### BACKGROUND OF THE INVENTION

Age-related macular degeneration, or AMD, is a degenerative condition of the macula in the center region of the retina of the human eye. Specifically, AMD blurs the sharp, central vision needed for "straight ahead" activities such as reading and driving an automobile. It happens that AMD is classified as either neovascular ("wet"), or non-neovascular ("dry"), AMD. Dry AMD, which is the most common form of the disease, occurs when the light sensitive cells in the macula slowly break down. Wet AMD, on the other hand, results from a leaking of blood and fluid under the macula of the eye, hence the term "wet" AMD. As a result of the increased fluid under the macula, the macula is lifted from its normal place at the back of the eye. Consequently, the macula is damaged as it is displaced.

Although wet AMD is far less prevalent than dry AMD, wet AMD is considered advanced AMD. Currently, the treatment options for wet AMD are limited, and no cure is available. With regard to the treatment of wet AMD, the first option available is photocoagulation. During the process of photocoagulation, a laser beam is directed to the leaky blood vessels to seal or destroy the blood vessels. Unfortunately, collateral damage to surrounding healthy tissue can be substantial with this surgical approach. Furthermore, this form of laser surgery is only available to a limited number of wet AMD patients, depending, in part, on the severity and stage of the disease.

A second treatment option for wet AMD is photodynamic therapy or "PDT". PDT involves marking a region of diseased retinal tissue with a chemical agent or "marking" agent. The marking agent is most often injected into the blood stream of a patient, wherein the marking agent transits the vasculature system of the patient and adheres to the diseased tissue. When subsequently illuminated by a laser light, the marking agent converts oxygen in a manner that causes the converted oxygen to kill the "marked" tissue.

The most common method for implementing photodynamic therapy, however, has a number of limitations. First, the marking of diseased tissue is often inexact. More particularly, some diseased areas may be missed by the marking agent while areas of healthy tissue may be inadvertently marked. Also, the illumination light typically used in photodynamic therapy has a wavelength of about 630nm. Using light at this wavelength results in a low absorption probability and an extensive depth of absorption (e.g. 2mm). Such a low absorption probability leads to an inefficient and incomplete killing of diseased tissue. Further, the extensive depth of absorption leads to the undesirable killing of healthy, as well as diseased, tissue. In addition to the limitations discussed above, the Point Spread Function ("PSF") for many laser systems is insufficient. The PSF may be defined as the finest volume of focus achievable for a given light beam, and for many laser systems the smallest PSF possible is on the order of 6 µm x 6 µm x 200 µm. Notably, a PSF of 6 µm x 6 µm x 200 µm is considered relatively large when compared to the average size of a region of AMD diseased tissue. Precise imaging and subsequent treatment of the marked region is therefore difficult. The impact of these limitations is that the traditional photodynamic therapy involves illuminating the entire retina for an extended period of time (e.g. 90 seconds). A consequence of this approach is that healthy as well as diseased retinal tissue is killed in areas where the marking agent is present.

Considering further the current state of the art for laser systems, the development of adaptive optics makes possible the very precise focusing of a laser beam into the eye of a patient. More specifically, with adaptive optics it is possible to reduce the PSF of a laser beam to about 2 µm x 2 µm x 20 µm. Precise focusing of the laser beam, in turn, provides for a higher concentration of laser energy in a smaller volume. More energy in a smaller volume leads to a more efficient and safer illumination of the retina. Furthermore, the concurrent development of ultra-fast, ultra-short pulse lasers as surgical tools has resulted in laser beams of greater wavelength being used to more efficiently illuminate smaller regions of retinal tissue. For example, femtosecond (*f*s) lasers, with wavelengths on the order of 800nm, are now being used in surgical procedures with greater frequency.

One application of adaptive optics and femtosecond lasers is Second Harmonic Generation ("SHG") imaging, as disclosed in issued U.S. Patent No. 7,510,283, titled "High Resolution Imaging for Diagnostic Evaluation of the Fundus of the Human Eye" by Bille, and assigned to the same assignee as the present invention. With SHG imaging, adaptive optics are used to focus a laser beam to a focal point in the eye having a PSF of about 2 µm x 2 µm x 20 µm. Due to the increased concentration of photons in a smaller volume of tissue, two red photons are absorbed in the corneal tissue and converted into a single blue photon. A plurality of blue photons constitute a response signal which is used to create an image of the corneal tissue.

A related advantage realized with the use of *f*s lasers and adaptive optics is a significant increase in the number of photons striking an illuminated region of tissue over a specified period of time. In the treatment of AMD, the periodicity with which photons strike a region of marked tissue impacts the effectiveness of the photodynamic treatment. For example, a single photon striking a marked region of diseased tissue may only have an electron state of about 1.5 eV. It happens, however, that an electron state of 1.5 eV is not sufficient to cause dye molecules to convert oxygen in a manner that will cause the destruction of tissue. If, however, two photons interact within a marking agent or "dye" molecule, within a very short interval of time (e.g. 10⁻¹³ sec), the effect of the two photons on the dye molecule becomes additive. This process is known as two photon excitation. When this happens, the additive effect of two photons interacting over a very short period of time creates an excited electron state of about 3 eV. Importantly, an electron state of 3 eV is adequate to cause the dye molecule to convert oxygen in a manner that kills the surrounding marked tissue.

The ability to create a reduced PSF for a laser beam (e.g. 2µm x 2µm x 20µm), along with the ability to generate very short duration laser pulses (e.g. ≅ 100 fs), allows two different optical phenomena to occur. These phenomena are: a) two-photon excitation fluorescence; and b) Second Harmonic Generation (SHG). Both of these independent phenomena result from the collision of two red photons in the PSF, and they may occur simultaneously at different locations. In the specific context of wet AMD, it has been shown that the two-photon excitation fluorescence phenomenon is useful for therapeutic purposes. On the other hand, due to the nature of wet AMD, it happens that the SHG phenomenon may also be used for diagnostic purposes.

With specific regard to SHG in the context of wet AMD, several additional factors are noteworthy. These factors include: a) the anatomical conditions at the site of diseased tissue in the retina; and b) the constituents that are used for making the marking agent that is used to identify the site. With regard to the first factor (anatomical conditions), as noted above, wet AMD is characterized by an accumulation of blood and fluid under the macula in the retina. In particular, this occurs because a so-called blood-brain barrier, that would otherwise prevent blood flow to the macula, is compromised by the diseased tissue. Stated differently, diseased tissue breaks down the blood-brain barrier, and this break-down allows blood to flow into, and to permeate the diseased tissue. Consequently, because the marking agent is carried in the blood stream of a patient, the marking agent can be carried to the blood accumulation site of the diseased tissue. This is not the case, however, with healthy tissue. Instead, the healthy tissue will preserve the blood-brain barrier, and thereby prevent the marking agent from flowing into the healthy tissue of the macula. Thus, the marking agent effectively marks only diseased tissue of the macula.

As noted above, it is possible for SHG to occur when a laser beam has very short duration pulses (≅ 100 fs), and the beam is focused to a very small PSF (2µm x 2µm x 20µm). There is, however, another factor that is of considerable importance for the SHG phenomenon to happen in the context of wet AMD. Specifically, this additional factor involves the need for there to be non-centro symmetric molecules present in the marking agent. For example, molecules of verteporfin are suitable for this purpose. In the event, it is the presence of the non-centro symmetric molecules that allow for the polarization effect of SHG to be useful as a diagnostic tool.

In review, with a blood-borne marking agent that includes non-centro symmetric molecules, these molecules can be carried by the marking agent through the vasculature and to the site of diseased tissue that is causing wet AMD. At the site of the diseased tissue, very short duration laser pulses can be focused and directed to a PSF in the marking agent. Two things then happen. For one, two-photon excitation fluorescence in the PSF will cause the marking agent to convert oxygen for killing the diseased tissue. For another, SHG in the PSF can occur when two red photons in a laser pulse simultaneously encounter a non-centro asymmetric molecule in the marking agent. It is this latter phenomenon that can be used to diagnostically identify where the oxygen conversion is occurring that will kill diseased tissue.

In light of the above, it is an object of the present invention to provide a system for treating age-related macular degeneration ("AMD"), specifically "wet" AMD. Another object of the present invention is to provide a system for treating wet AMD which utilizes adaptive optics and an ultra-fast, ultra-short pulse laser to induce two-photon excitation for photodynamic therapy. Yet another object of the present invention is to provide a system for treating wet AMD that includes the precise imaging of a region of diseased tissue. Another object of the present invention is to provide a method for treating "wet" AMD that incorporates a marking agent with non-centro symmetric molecules, that provides an SHG response in the PSF at the focal point of a laser beam, for visualizing the location of the PSF, while simultaneously causing the marking agent to kill diseased tissue in the PSF. Still another object of the present invention is to provide a system for treating wet AMD that minimizes collateral damage to surrounding healthy retinal tissue during PDT. Yet another object of the present invention is to provide a system for treating wet AMD that is easy to use, relatively simple to manufacture and comparatively cost effective.

### SUMMARY OF THE INVENTION

A system for treating the disease of age-related macular degeneration ("AMD") in the retina of a human eye includes a chemical or "marking" agent for marking a region of diseased retinal tissue. One such marking agent is verteporfin. Additionally, the system of the present invention includes a laser source for generating a laser beam. Preferably, the laser beam is a femtosecond laser beam, having a wavelength of about 800nm, a pulse duration in the range of about 200-800 femtoseconds, and a pulse energy of about 1 nJ. Working in concert with the laser source is an optical assembly for directing and focusing the laser beam to a focal point in the region of diseased retinal tissue. Additionally, the optical assembly may include a wavefront sensor for detecting an alignment of the optical axis of the eye. In any event, the optical assembly will include adaptive optics. More specifically, the adaptive optics of the optical assembly include: a scanning unit for moving the laser beam between adjacent focal points in the region of diseased tissue; an active mirror for compensating the laser beam and directing the beam into the scanning unit; and, a plurality of focusing lenses for focusing the laser beam to the focal point in the diseased retinal tissue.

For the purposes of the present invention, the active mirror is preferably of the type disclosed in U.S. Patent No. 6,220,707, entitled "Method for Programming an Active Mirror to Mimic a Wavefront" issued to J. Bille. As contemplated by the present invention, the active mirror is positioned on the beam path to compensate the laser beam as the beam is reflected off the mirror and directed toward the scanning unit. As can be appreciated by the skilled artisan, compensation of the laser beam is required to account for the aberrations introduced into the beam as the beam transits the eye. More specifically, compensation is required to minimize the individual phase shift deviations that affect each contiguous ray of light as the laser beam strikes the eye at some predetermined angle, and subsequently passes through the cornea. A computer controller, which is in electronic communication with both the laser source and the optical assembly, directs the movement of the individual facets of the active mirror to thereby compensate the beam.

In addition to the laser source and optical assembly disclosed above, the system of the present invention includes an imaging unit for creating an image of the diseased tissue. A response signal, generated by Second Harmonic Generation ("SHG") imaging, is used to create the image. Further, a beam splitter is optically aligned with the imaging unit for directing the response signal into the imaging unit. The computer controller is in electronic communication with the imaging unit for receiving and processing image data.

In the operation of the present invention, an image of the region of diseased retinal tissue is created using SHG imaging. Specifically, the wavefront sensor verifies the alignment of the optical axis as the laser beam is directed to a focal point in the region of diseased tissue. As envisioned by the present invention, the focal point has a PSF of approximately 2µm x 2µm x 20µm. As the laser beam illuminates the focal point, a response signal is generated which is used by the imaging unit to create an image of the diseased tissue. The image is subsequently communicated electronically to the computer controller, after which time the data is used to more precisely focus the laser beam during a subsequent PDT treatment.

Once the imaging of the diseased tissue is complete, the marking agent is introduced into the bloodstream of the patient, often by injecting the marking agent into the arm of the patient. After injection, the marking agent transits the vascular system of the patient to collect in those areas of the retina damaged by AMD, thereby marking those areas for treatment. Following the imaging and marking of the diseased tissue, the laser beam is focused onto a focal point in the volume of diseased tissue. Specifically, the laser beam is directed along the beam path to reflect off the active mirror. As disclosed above, the active mirror compensates the laser beam and directs the beam toward the scanning unit. After reflecting off the active mirror, the laser beam transits the scanning unit and the focusing lenses, wherein the laser beam is focused to the focal point in the retina. After focusing the laser beam to an initial focal point, the scanning unit moves the beam to illuminate a plurality of focal points according to a predetermined scanning pattern. More specifically, each focal point is illuminated with about five femtosecond laser pulses at a rate of about 1 pulse/10⁻¹³ seconds. At this rate of illumination, and given the high concentration of photons in a relatively small PSF, two-photon excitation occurs. During two-photon excitation, the dye molecules of the marking agent convert oxygen in a manner that causes the oxygen to kill the diseased tissue. As the scanning of the beam continues, the dye molecules continue to convert oxygen thereby killing more of the diseased tissue. Illumination continues until the region of diseased tissue is effectively destroyed. It can be appreciated that the system of the present invention, as disclosed above, ensures that a smaller volume of diseased retinal tissue is effectively illuminated and treated without adversely affecting the surrounding healthy tissue.

In another aspect of the present invention, both the imaging and the treatment functions of the system and method are accomplished simultaneously. In this case, a marking agent that includes non-centro symmetric molecules is provided. This marking agent is then introduced into the blood stream of a patient, and is allowed to permeate the diseased tissue. Anatomically, this can occur in the case for "wet" AMD, when the blood-brain barrier in the eye is compromised by the diseased tissue.

Once the diseased tissue has been permeated with the marking agent, a pulsed laser beam is focused to a focal spot within the diseased tissue to kill the diseased tissue. To do this, each individual pulse in the laser beam is generated to have a pulse duration that is less than about 150 fs, and adaptive optics are used to create a focal spot that is characterized by a point spread function (PSF) which is approximately 2µm x 2µm x 20µm in size.

As intended for the present invention, the imaging and treatment of diseased tissue is caused to occur simultaneously at the PFS. In detail, due to the characteristics of pulses in the laser beam, and the size of the PSF, photons in a same laser pulse may cause either Second Harmonic Generation (SHG) or two photon excitation fluorescence. Specifically, photons that interact with a non-centro symmetric molecule of the marking agent will generate a Second Harmonic Generation (SHG) response signal. On the other hand, photons in the laser pulse may interact for a two-photon excitation fluorescence in the PSF to create an excited electron state. In the event, detecting the SHG response signal will verify a positioning of the focal spot (PSF) in the diseased tissue, and the excited electron state caused by two-photon excitation fluorescence will induce the marking agent to convert oxygen and thereby kill diseased tissue.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a schematic view of the system of the present invention showing the interrelationships of the system components;
Fig. 2 is a representative illustration of a three-dimensional focal point in a region of diseased and marked retinal tissue;
Fig. 3 is a representative illustration of a top view of a focal point in a region of diseased and marked retinal tissue; and
Fig. 4 is a representative illustration of a three-dimensional focal point in a region where only diseased tissue is marked.

### DESCRIPTION OF THE PREFERRED EMBODIMENTS

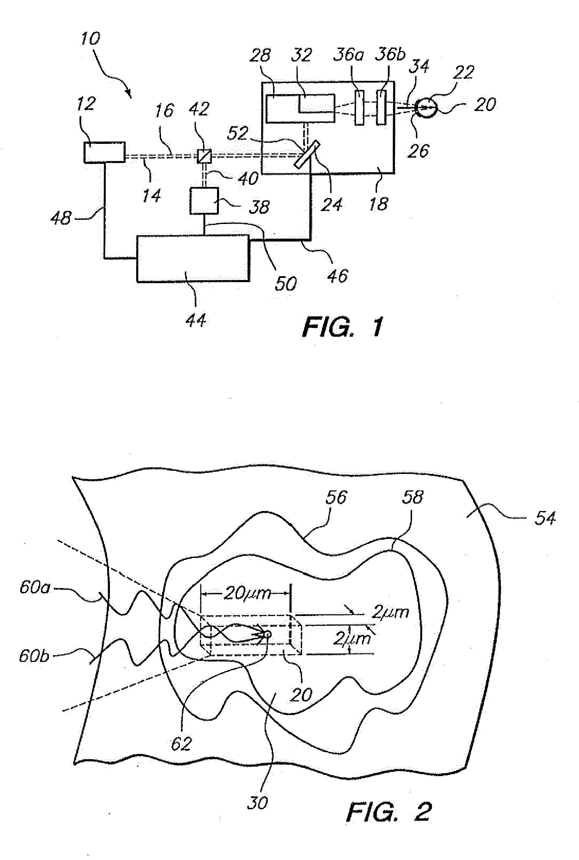

A system in accordance with the present invention is shown in Fig. 1 and is generally designated 10. As shown, the system 10 includes a laser source 12 for directing a laser beam 14 along a beam path 16. Specifically, the laser source 12 is a tunable, femtosecond (f s) laser source 12. More specifically, the laser source 12 generates a laser beam 14 having a wavelength of about 800nm, a pulse duration in a range of about 200-800 femtoseconds, and a pulse energy of about 1 nJ.

Working in concert with the laser source 12 is an optical assembly 18, for focusing the laser beam 14 onto a focal point 20 in the eye 22. As contemplated by the present invention, the optical assembly 18 includes adaptive optics for more precisely focusing the laser beam 14. More specifically, the optical assembly 18 includes an active mirror 24 optically aligned with the laser source 12 for compensating the laser beam 14 as the beam 14 reflects off the mirror 24. As can be appreciated by the skilled artisan, the active mirror 24 must compensate the laser beam 14 for aberrations introduced into the beam 14 as the beam 14 transits the cornea 26 of the eye 22. Stated differently, the active mirror 24 must compensate the laser beam 14 by minimizing the individual phase shift deviations that adversely affect each contiguous ray of light as the laser beam 14 transits the cornea 26. Compensation, in turn, allows the laser beam 14 to be focused to a smaller focal point 20 in the eye 22, thereby leading to a higher concentration of light in a smaller volume of tissue.

Still referring to Fig. 1, the optical assembly 18 also includes a scanning unit 28 for moving the laser beam 14 between a plurality of focal points in a region of diseased tissue 30 (Fig. 2). It can be appreciated that the scanning unit 28 may be any of a type well known in the pertinent art that is capable of focusing the laser beam 14 along a predetermined beam path 16. As shown in Fig. 1, the scanning unit 28 is optically aligned with the active mirror 24 for receiving the laser beam 14 as the beam 14 reflects off the mirror 24. Preferably, the optical assembly 18 also includes a wavefront sensor 32 for detecting the alignment of an optical axis 34 of the eye 22 prior to the imaging and subsequent treatment of the region of diseased tissue 30. In addition to the scanning unit 28 and wavefront sensor 32, the optical assembly 18 includes a plurality of focusing lenses, of which lenses 36a and 36b are only exemplary. The lenses 36a and 36b are optically aligned with the scanning unit 28 for focusing the laser beam 14 onto the focal point 20 in the cornea 26.

As contemplated by the present invention, the system 10 includes an imaging device 38 for receiving and processing a return signal 40 generated during a Second Harmonic Generation imaging of the diseased tissue 30. Further, a beam splitter 42 is optically aligned with the active mirror 24 and the imaging unit 38 for directing the return signal 40 into the imaging unit 38. As further shown in Fig. 1, a computer controller 44 is in electronic communication with the optical assembly 18, the laser source 12, and the imaging unit 38 via electrical cables 46, 48 and 50 respectively.

In addition to the elements of the present invention disclosed above, an important aspect of the present invention is a chemical or "marking" agent (not shown) for marking the regions of diseased tissue 30. In one embodiment of the present invention, the marking agent is verteporfin. It can be appreciated that the marking agent may be introduced into the bloodstream of the patient (not shown), for transiting the vasculature of the patient and entering the eye 22 through the optical nerve.

In the operation of the present invention, the system 10 of the present invention is first used to generate images of the region of diseased tissue 30 using SHG imaging. Specifically, the laser source 12 generates a femtosecond laser beam 14 which is directed toward the optical assembly 18, and more specifically toward the active mirror 24. It is to be understood that the active mirror 24 is programmed by the computer controller 44 to compensate the laser beam 14 as the laser beam 14 reflects off of the surface 52 of the mirror 24. Importantly, for the active mirror 24 to be properly programmed, the computer controller 44 must know the exact alignment of the optical axis 34 of the eye 20. Preferably, the wavefront sensor 32 provides the necessary alignment data. After the mirror 24 is programmed, the laser beam 14 reflects off the mirror 24 and transits the scanning unit 28, subsequently exiting in the direction of the focusing lenses 36a and 36b. As the laser beam 14 transits the focusing lenses 36a and 36b, the laser beam 14 is focused onto the desired focal point 20 in the region of diseased tissue 30. Through the use of the adaptive optics of the optical assembly 18, the laser beam 14 is precisely focused to the focal point 20 with a PSF of about 2µm x 2µm x 20µm (Fig. 2). The laser beam 14 illuminates the region of diseased retinal tissue 30, and a response signal 40 is generated. The response signal 40, in turn, travels back through the optical assembly 18 and is directed by the beam splitter 42 into the imaging unit 38. As contemplated by the present invention, the image data generated by the imaging unit 38 is transmitted to the computer controller 44, wherein the data is used to verify the location and size of the region of diseased retinal tissue 30.

Once the SHG imaging of the region of diseased tissue 30 has been completed, the marking agent is introduced into the blood stream of the patient. As envisioned by the present invention, the marking agent enters the eye 22 and collects in the retina 54. As can be appreciated by referring to Fig. 2, the marking agent outlines a region of tissue (defined by line 56) that includes the region of diseased tissue 30. The outer limits of the region of diseased tissue 30 are defined by line 58. As shown in Fig. 2, there are areas of healthy tissue, specifically those areas of tissue between lines 56 and 58, that are inadvertently marked by the marking agent. Due to this "overlapping" by the marking agent, it is possible that healthy tissue may be inadvertently destroyed if illuminated by the laser beam 14. Therefore, the optical assembly 18 is used to precisely focus the laser beam 14 to a focal point 20 for PDT treatment, in much the same manner as the optical assembly 18 is used to focus the laser beam 14 for imaging.

Considering now the PDT treatment in greater detail, a femtosecond laser beam 14 as disclosed above is focused onto the focal point 20 in the retina 54 of the eye 22. As shown in Fig. 2, the laser beam 14 may be represented as a series of red photons, of which photons 60a and 60b are exemplary. Through the use of the femtosecond laser source 12 and the optical assembly 18 of the present invention, the concentration or number of red photons (e.g. 60a and 60b) striking the focal point 20 in the retina 54 over a given time period is increased significantly. Importantly, with an increased concentration of red photons 60a and 60b illuminating the focal point 20, it can happen within a very short interval of time (e.g.10⁻¹³sec.) that two photons 60a and 60b will interact together with a single dye molecule 62 of the marking agent. When this two-photon interaction occurs, the effect of the two photons 60a and 60b striking a single dye molecule 62 becomes additive. Stated differently, although each photon 60a and 60b alone has an electron state of about 1.5 eV, the additive effect of both photons 60a and 60b is to create an excited electron state of about 3 eV. It happens that an electron state of 1.5eV is insufficient to induce the oxygen conversion needed to kill the region of diseased tissue 30. An excited electron state of 3eV, however, is sufficient to cause the desired effect between the dye molecule 62 and the surrounding diseased tissue 30, i.e. oxygen conversion that kills the diseased tissue 30. It is to be appreciated that the two-photon 60a and 60b excitation of the present invention yields a very high probability of energy absorption in a very thin layer of the diseased tissue 30, e.g. within a depth of about five microns. Accordingly, very small volumes of diseased tissue within the focal point 20 can be precisely illuminated and killed in three dimensions. Additionally, collateral damage to regions of healthy tissue is minimized.

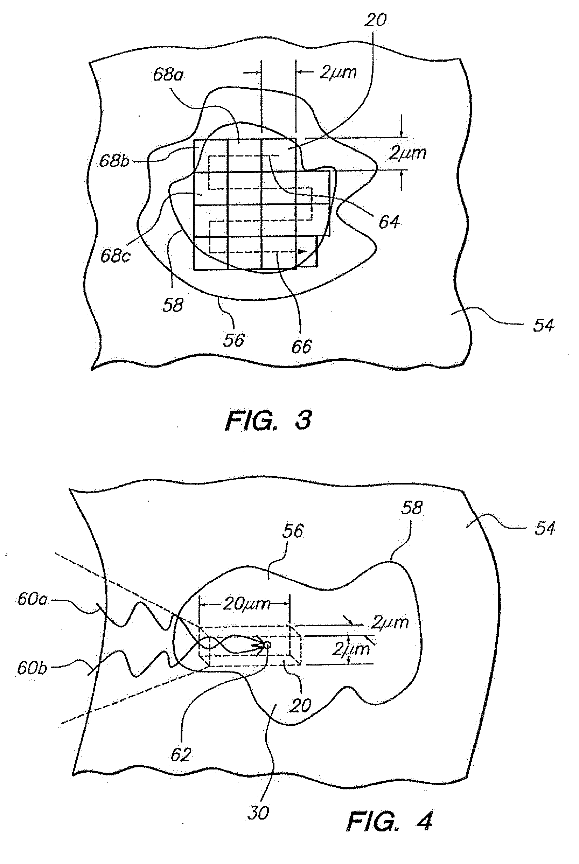

Referring now to Fig. 3, a top view of the region of diseased tissue 30, as viewed along the beam path 16 is presented. As contemplated by the present invention, the optical assembly 18 focuses the laser beam 14 to a start point 64 within the region of diseased tissue 30. According to a scanning sequence 66 transmitted by the computer controller 44, the scanning unit 28 moves the laser beam 14 sequentially from an initial focal point 20 to a series of adjacent focal points, of which 68a, 68b and 68c are exemplary. More specifically, each focal point is illuminated with about five femtosecond laser pulses at a rate of about 1 pulse/10⁻¹³ seconds. As contemplated by the present invention, the scanning sequence 66 continues until the region of diseased tissue 30 is effectively killed.

In Fig. 4 a condition is shown wherein a region of diseased tissue 30 is coincident with the marked region 56 of the retina 54. As noted earlier, this will happen when the blood-brain barrier of an eye 22 is compromised by the region of diseased tissue 30. This coincident condition, together with the incorporation of non-centro symmetric molecules into the agent that marks the region 56, allows for the imaging of the marked region 56, and the killing of tissue in the region of diseased tissue 30, to be accomplished simultaneously. For these disparate purposes, the pulsed laser beam 14 is focused to a focal spot 20 within the region of diseased tissue 30. In detail, this focal spot 20 is characterized by a point spread function (PSF) that is approximately 2µm x 2µm x 20µm in size. Preferably, individual pulses in the laser beam 14 will have a pulse duration less than about 150 fs, and the wavelength "λ" of the laser beam 14 will be approximately 800 nm.

As envisioned for the present invention, an interaction of photons 60a and 60b in a pulse of the laser beam 14, in the focal point (PSF) 20, can result in either of two independent phenomena. For one, the photons 60a and 60b can interact with a non-centro symmetric molecule (e.g. dye molecule 62) in the PSF 20 to generate a Second Harmonic Generation (SHG) response signal. This response signal can then be used by the imaging unit 38 to identify and verify the location of the PSF 20 as being within the region of diseased tissue 30. For another, the photons 60a and 60b can interact with each other to create the excited electron state (two-photon excitation fluorescence) that will convert oxygen in the marked region 56 and kill diseased tissue in the region of diseased tissue 30. For the present invention, all of this can be done simultaneously.

While the particular Diagnostic Imaging for Age-Related Macular Degeneration (AMD) Using Second Harmonic Generation (SHG) Techniques as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system for performing a therapeutic treatment of diseased tissue which comprises:
a means for introducing a marking agent into the blood stream of a patient to permeate the diseased tissue with the marking agent, wherein the marking agent includes non-centro symmetric molecules; and
a means for focusing a pulsed laser beam to a focal spot within the diseased tissue, wherein individual pulses in the laser beam have a pulse duration less than about 150 fs and the focal spot is **characterized by** a point spread function (PSF) approximately 2µm x 2µm x 20µm in size, and wherein an interaction of photons in a laser pulse with a non-centro symmetric molecule of the marking agent in the PSF generates a detectable Second Harmonic Generation (SHG) response signal to verify a position of the focal spot in the diseased tissue, and the interaction of photons in the laser pulse enables a two-photon excitation fluorescence in the PSF to create an excited electron state, wherein the excited electron state induces the marking agent to convert oxygen to kill the diseased tissue.

2. A system as recited in claim 1 wherein the diseased tissue is macula in a retina of a patient.

3. A system as recited in claim 1 or 2 wherein the wavelength of the laser beam "λ" is approximately 800 nm.

4. A system as recited in claim 1, 3 or 3 wherein the focal spot (PSF) of the laser beam is moved to a plurality of focal spots, in sequence, through the diseased tissue.

5. A system as recited in any preceding claim wherein the diseased tissue is macula in a retina of a patient, and wherein the wavelength of the laser beam "λ" is approximately 800 nm.

6. A method for diagnostically identifying diseased tissue, during a therapeutic treatment of the diseased tissue, which comprises the steps of:
introducing a marking agent into the blood stream of a patient, wherein the marking agent includes non-centro symmetric molecules;
allowing the marking agent to permeate the diseased tissue;
focusing a pulsed laser beam to a focal spot within the diseased tissue, wherein individual pulses in the laser beam have a pulse duration less than about 150 fs and the focal spot is **characterized by** a point spread function (PSF) approximately 2µm x 2µm x 20µm in size, and wherein an interaction of photons in a laser pulse with a non-centro symmetric molecule of the marking agent in the PSF generates a Second Harmonic Generation (SHG) response signal; and
detecting the SHG response signal to verify a positioning of the focal spot in the diseased tissue.

7. A method for therapeutic treatment of a diseased tissue, which comprises the steps of:
providing a marking agent, wherein the marking agent includes non-centro symmetric molecules;
introducing the marking agent into the blood stream of a patient;
allowing the marking agent to permeate the diseased tissue;
focusing a pulsed laser beam to a focal spot within the diseased tissue, wherein individual pulses in the laser beam have a pulse duration less than about 150 fs and the focal spot is **characterized by** a point spread function (PSF) approximately 2µm x 2µm x 20µm in size;
causing photons in a laser pulse to interact with a non-centro symmetric molecule of the marking agent in the PSF to generate a Second Harmonic Generation (SHG) response signal;
detecting the SHG response signal from the causing step to verify a positioning of the focal spot in the diseased tissue; and
enabling photons in the laser pulse to interact for a two-photon excitation fluorescence in the PSF to create an excited electron state, wherein the excited electron state induces the marking agent to convert oxygen to kill the diseased tissue.

8. A method as recited in claim 6 or 7 wherein the non-centro symmetric molecule is verteporfin.

9. A method as recited in claim 6, 7 or 8 wherein the diseased tissue is macula in a retina of a patient.

10. A method as recited in any of claims 6 to 9 wherein the wavelength of the laser beam "λ" is approximately 800 nm.

11. A method as recited in any of claims 6 to 10 wherein the wavelength of the SHG signal response "λₛ" is 400 nm.

12. A method as recited in any of claims 6 to 11 wherein the non-centro symmetric molecules provoke the SHG response signal.

13. A method as recited in any of claims 6 or 8 to 12 further comprising the step of creating an excited electron state with two-photon excitation fluorescence in the PSF to convert oxygen in the marking agent and kill the diseased tissue in the PSF.

14. A method as recited in any of claims 6 to 13 wherein the detecting step and the creating step are accomplished simultaneously.

15. A method as recited in any of claims 6 to 14 further comprising the step of moving the focal spot of the laser beam to a plurality of focal spots, in sequence, through the diseased tissue.

16. A method as recited in any of claims 6 to 14 wherein the allowing step is accomplished as a consequence of a compromise of a blood-brain barrier in the eye of a patient.
